# EUROPÄISCHE PATENTSCHRIFT

(11) **EP 0 925 032 B1**
(45) Veröffentlichungstag und Bekanntmachung des Hinweises auf die Patenterteilung: **28.01.2004**
(21) Anmeldenummer: 98928322.1
(22) Anmeldetag: 27.05.1998
(51) Int. Cl.: A61B 17/15

(54) **KNOCHENSÄGELEHRE**
BONE SAWING JIG
GABARIT DE SCIAGE D'OS

(30) Priorität: 27.05.1997 DE 29709287 U; 12.11.1997 DE 29720105 U
(43) Veröffentlichungstag der Anmeldung: 30.06.1999
(73) Patentinhaber: WALDEMAR LINK GmbH & Co. KG, 22339 Hamburg (DE)
(72) Erfinder: KELLER, Arnold, D-23863 Kayhude (DE)
(74) Vertreter: Glawe, Delfs, Moll & Partner
(86) Internationale Anmeldenummer: PCT/EP1998/003132
(87) Internationale Veröffentlichungsnummer: WO 1998/053747

(56) Entgegenhaltungen:
- EP-A- 0 661 023
- WO-A-94/08528
- DE-U- 9 301 611
- FR-A- 2 635 675
- FR-A- 2 672 489
- US-A- 4 457 307
- US-A- 4 703 751
- US-A- 5 122 144

## Beschreibung

Beim Ersatz der oberschenkelseitigen Kniegelenkgleitflächen durch schalenförmige Prothesen strebt man eine sparsame Resektion an. Den natürlichen Verlauf der Gelenkflächen nähert man durch eine Folge von Facettenflächen an, die durch ebene Sägeschnitte erzeugt werden. Damit sie insbesondere bei zementloser Implantation genau mit der knochenseitigen Kontur des Implantats übereinstimmen, müssen die Sägeschnitte genau geführt werden. Zu diesem Zweck bedient man sich einer Sägelehre, welche Führungsschlitze für die Säge enthält, die entsprechend der gewünschten Facettenfolge angeordnet sind.

Dabei ist es wichtig, daß sämtliche Facettenschnitte mit ein und derselben Sägelehre durchgeführt werden können, die zwischen aufeinanderfolgende Schnitten nicht umgesetzt zu werden braucht und die auch eine gute Beobachtung des Resektionsbereichs gestattet. Dies wird bei einer bekannten Sägelehre (DE-U 93 01 611) dadurch ermöglicht, daß die Sägelehre zwei Seitenplatten aufweist, zwischen denen eine Mehrzahl von zur Führung eines Sägeblatts in sämtlichen Schnittebenen bestimmten Stangenpaaren gehalten ist. In den Seitenplatten sind Bohrungen vorgesehen, durch die Fixierstifte mit unterschiedlicher Schrägung eingebracht werden können, die dazu bestimmt sind, die Sägelehre in der gewählten Position an dem zu bearbeitenden Knochen zu fixieren. Diese Sägelehre hat den Nachteil, daß sie zum Ansetzen eines Probeimplantats an die Facettenfläche gänzlich entfernt werden muß. Wenn sich eine Nachresektion als notwendig erweist, muß sie wieder angesetzt werden, wobei es nicht leicht ist, die ursprüngliche Positionierung genau wieder zu treffen. Unmöglich ist es, eine Position zu finden, die mit der ursprünglichen Position in Längsrichtung des Knochen genau fluchtet, aber im Verhältnis zu diesem im Sinne einer etwas stärkeren Resektion parallel verschoben ist.

Ferner ist eine Fräslehrenanordnung bekannt (EP-A 661 023), die aus einer Fräslehre, einem Positionierteil und einem Fixierteil besteht. Der Fixierteil wird von zwei beiderseits mit der Fräslehre bzw. dem Positionierteil lösbar verbindbaren Seitenwangen gebildet. Um die richtige Position der Anordnung am Knochen zu finden, wird zunächst der Positionierteil angesetzt, danach werden die Seitenwangen mit diesem verbunden und am Knochen fixiert. Danach wird der Fixierteil abgenommen und durch die Fräslehre ersetzt, die nun von den Seitenwangen in der richtigen Position am Knochen gehalten ist. Wenn die Resektion der Facettenflächen vollendet ist, werden die Fräslehre und die Seitenwangen abgenommen, und es wird ein Probeimplantat an die Facettenflächen angesetzt, um die Passung zu überprüfen. Sollte sich dabei herausstellen, daß die Facettenflächen einer Korrektur bedürfen, muß die Fräslehre wieder angesetzt werden, und zwar in einer korrigierten Position, die genau auf die bereits vorhandenen Resektionsflächen ausgerichtet sein muß. Zu diesem Zweck umfaßt die bekannte Anordnung ein zusätzliches Führungsstück, das an die vorhandenen Resektionsflächen angesetzt wird, um die neue Positionierung der Fräslehre und deren Seitenwangen zu ermöglichen. Da durch die Abmessungen dieses zusätzlichen Führungsstücks bestimmt wird, welchen Abstand die neu zu fertigenden Facettenflächen von den vorhandenen haben, und weil dieser Abstand je nach Lage des Falles unterschiedlich sein kann, müssen offenbar mehrere derartige zusätzliche Führungsstücke vorgesehen werden. Dies ist aufwendig. Aufwendig ist auch das Positionierverfahren, weil die zum Fixieren verwendeten Seitenwangen abgenommen und in neuer Position wieder angesetzt werden müssen. Schließlich besteht ein Nachteil dieses Instruments darin, daß das Bearbeitungsfeld nur sehr begrenzt optisch kontrolliert werden kann, weil die Teile der Fräslehre flächig ausgebildet sind. Dies hängt damit zusammen, daß die Art der Werkzeugführung bei einer Fräslehre anders als bei einer Sägelehre ist. Es fehlen demzufolge auch Seitenplatten, zwischen denen Stangenpaare gehalten sind.

Bekannt ist auch eine Sägelehre (US-A-5,122,144) die einerseits an einer Stange fixiert wird, die in den intramedulären Kanal eingeschoben wird, und andererseits durch eine Seitenwange gehalten wird, die lösbar am Außenrand der Sägelehre angeschraubt wird und mittels mehrerer Stifte an den Femurkondülen fixiert wird. Sobald die Sägeschnitte vollzogen sind, wird zunächst die Seitenwange und anschließend die Sägelehre entfernt. Vorkehrungen für ein wiederholtes Ansetzen der Sägelehre oder eine Neujustierung derselben sind nicht getroffen.

Schließlich ist eine Sägelehre bekannt (WO94/08528), die einen an die Diaphyse des Femurs anzuklemmenden Fixierteil umfaßt, an welchem zunächst ein Positionierteil, später eine Sägelehre zur Führung eines Sagittalschnitts und danach eine Sägelehre zur Führung der Facettenschnitte angebracht werden können. Die Anbringung geschieht über eine Längsführung mit Maßeinteilung, die es gestattet, die verschiedenen Sägelehren in unterschiedlicher Längspositionierung anzusetzen. Die Notwendigkeit, den Fixierteil in Kombination mit mindestens drei Ansatzstücken zu verwenden, hat den Nachteil, daß Fehler durch Vertauschung oder Fehleinstellung provoziert werden. Der Aufbau ist wesentlich verschieden von denjenigen anderen oben erörterten Typen von Resektionslehren.

Ausgehend von dem oben an erster Stelle geschilderten Stand der Technik (DE-U 93 01 611) sucht die Erfindung eine Sägelehre zu schaffen, die das Ansetzen eines Probeimplantats und die Verstellung der Sägelehre im Falle einer Korrektur der Resektionsflächen ermöglicht. Dies gelingt durch die in den Ansprüchen angegebene Merkmalskombination.

Dank diesen Merkmalen brauchen die Seitenwangen zum Testen des Implantats nicht vom Knochen abgenommen zu werden. Sie bleiben während des Tests an Ort und Stelle und sind so ausgeführt, daß das Probeimplantat oder die Probeschablone währenddessen an die Resektionsflächen angesetzt werden können. Sollte es sich als notwendig erweisen, daß die Facettenflächen nachbearbeitet werden, so wird die Sägelehre erneut an die in der alten Position verbliebenen Seitenwangen angesetzt. Die Verstellbarkeit in Längsrichtung gestattet es nun, die Stellung der Sägelehre so zu wählen, wie es dem Ausmaß der gewünschten Korrektur entspricht. Die Verstellbarkeit sollte aus diesem Grunde stufenlos möglich sein. Aber auch eine stufige Verstellbarkeit genügt den praktischen Erfordernissen in vielen Fällen. Eine Vertauschung von Komponenten ist ausgeschlossen, weil nur eine Komponente, nämlich der Sägelehrenteil gelöst und ggf. wieder angesetzt werden muß.

Alle Facettenschnitte können durchgeführt werden, ohne daß die Sägelehre umgesetzt werden muß.

Nach einem weiteren Merkmal der Erfindung kann vorgesehen sein, daß eine zur Anlage an den distalen Condylenflächen des Femurs bestimmte Anschlagfläche der Sägelehre in Längsrichtung verstellbar oder entfernbar ist. Die Anschlagfläche bestimmt in ihrer Normalstellung das Ausmaß der Resektion in normalen Fällen. Durch die Verstellbarkeit oder Entfernbarkeit der Anschlagfläche kann das Ausmaß der Resektion von vornherein abweichend von dem üblichen Resektionsmaß verändert werden, und zwar sowohl in positiver als auch negativer Richtung.

Da die Erfindung dem Operateur die Möglichkeit gibt, sein Vorgehen je nach dem augenblicklichen Befund gegebenenfalls zu ändern, ist eine Ausführung der Sägelehre besonders vorteilhaft, die ihm größtmöglichen optischen Überblick über das Resektionsgebiet gibt. Aus diesem Grunde wird die erfindungsgemäße Sägelehre zweckmäßigerweise weitgehend blickdurchlässig gebildet, indem allen Stangen, die die Sägeblattführung bilden, verhältnismäßig dünn sind, d.h. daß ihr Längen/Durchmesser-Verhältnis mindestens 10 beträgt. Dies ist an sich bekannt (DE-U-93 01 611, FR-A-26 72 489).

Wenn im vorliegenden Zusammenhang von der Längsrichtung gesprochen wird, so ist damit die Längsrichtung der Sägelehre gemeint. Diese wird bestimmt durch Bezugnahme auf die Längsrichtung der Diaphyse des Knochens und stimmt in der Regel mit dieser etwa überein.

Die Erfindung wird im folgenden näher unter Bezugnahme auf die Zeichnung erläutert, die ein vorteilhaftes Ausführungsbeispiel veranschaulicht. Es zeigen:

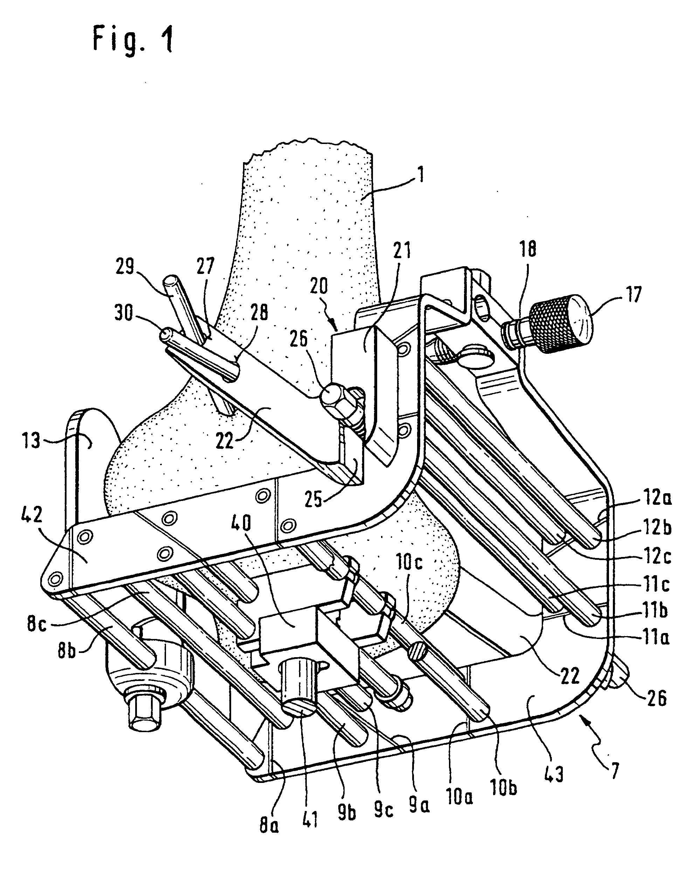

- Fig. 1: eine perspektivische Gesamtansicht

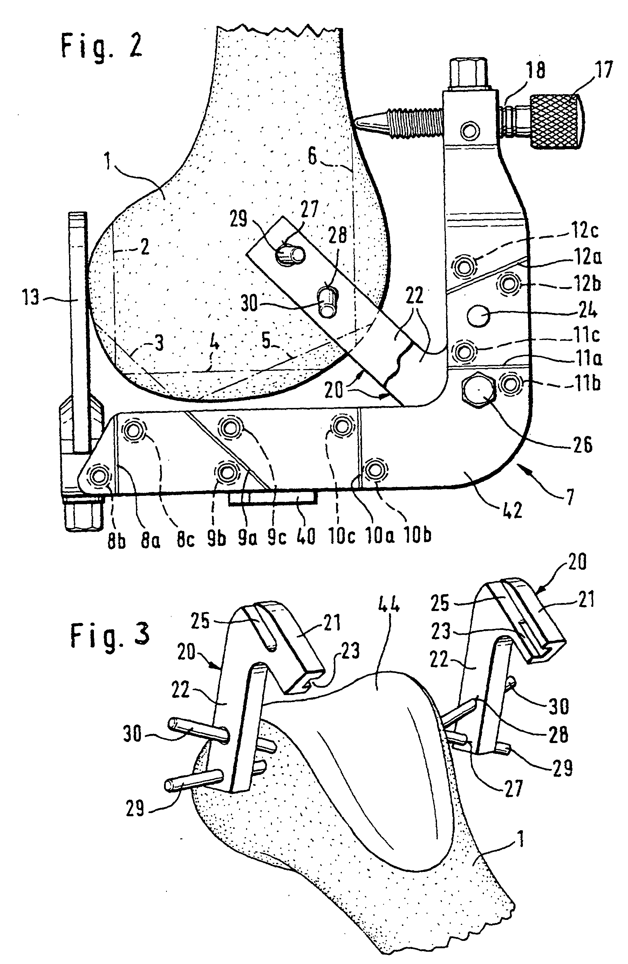

- Fig. 2: eine Seitenansicht mit weggenommener vorderer Seitenwange und
- Fig. 3: eine perspektivische Ansicht des Knochens mit abgenommener Sägelehre und am Knochen verbleibenden Seitenwangen..

Die Condylen 1 des Oberschenkelknochens sind gemäß der Innenkontur einer nicht dargestellten Endoprothese in den strichpunktiert angedeuteten Ebenen 2, 3, 4, 5 und 6 zu resezieren. Zur Führung des Sägeblatts dient eine Sägelehre 7, die aus zwei Seitenplatten 42, 43, besteht, die durch Rundstangen 8-12 miteinander starr verbunden sind. Diese Rundstangen bilden paarweise die Führungen für das Sägeblatt. Die Führungsrichtungen sind auf den Seitenplatten 42, 43 markiert durch Striche 8a, 9a, 10a, 11a und 12a. Diesen Markierungsstrichen sind die Führungsstangen 8b,c; 9b,c; 10b,c; 11b,c und 12b,c zugeordnet.

Die Stangen jedes Paares sind zueinander versetzt angeordnet; und zwar ist jeweils diejenige Stange mit dem Index b, die auf der Seite des zu entfernenden Resektats liegt, weiter vom Knochen entfernt als die auf der Seite des verbleibenden Knochens liegende Stange mit dem Index c. Wie man anhand der Zeichnung leicht erkennt, wird dadurch die Resektionsebene so festgelegt, daß die Säge sich über diese Ebene hinaus dem verbleibenden Knochenteil nicht weiter nähern kann. Sie kann sich aber davon weiter entfernt einstellen. Ein Schnitt im Sinne zu kräftiger Resektion wird dadurch ausgeschlossen, solange die Lehre unbewegt am Knochen fixiert ist. Ermöglicht wird hingegen ein mehrstufiger Schnitt, bei dem die Säge zunächst in einer gewissen Entfernung von der angestrebten Resektionsebene geführt wird, um einen Rohschnitt durchzuführen, dem ein Feinschnitt in der Resektionsebene folgt.

Die Ausführung der Sägeschlitze aus Stangen vergrößert das Sichtfeld und gestattet es dem Chirurgen, die Wirkung seines Schnitts ständig optisch zu überprüfen, was bei plattenförmiger Ausbildung des Lehrenkörpers nicht möglich ist. Die Dicke der Stangen beträgt beispielsweise 5 oder 6 mm bei einer Länge von 80-90 mm.

Im allgemeinen wird der besseren Übersicht halber eine Ausführung beider Schlitzbegrenzungen als Stangen bevorzugt. Jedoch ist dies nicht unbedingt erforderlich. Beispielsweise könnten die im dargestellten Beispiel von den Stangen 9c und 10c gebildeten Schlitzbegrenzungen von den beiden Kanten einer und derselben Platte gebildet sein. Dennoch hätte der Chirurg dank der Stangenausführung der jeweils anderen Schlitzbegrenzung einen gegebenenfalls noch hinreichenden Blick auf den Resektionsbereich..

Die Sägelehre weist eine dorsale Anschlagplatte 13 auf, die im allgemeinen zur Einstellung der Lehre am Knochen an den dorsalen Gleitflächenteilen der Condylen 1 in Anlage gebracht wird.. Sie umfaßt ferner einen frontalen Teil 16, der normalerweise einen gewissen Abstand von den Condylen hat. Die darin enthaltene Schraube 17 dient nicht zum Fixieren der Lehre am Knochen (obwohl sie dafür verwendet werden kann), sondern ihre Spitze dient zum Anzeigen derjenigen Stelle, an der die ventrale Resektionsfläche 6 oben enden soll. Sie kann mit einer Maßeinteilung 18 versehen sein, die eine Bewertung ermöglicht, ob die gewählte Sägelehre und die ihr entsprechende Prothese für den in Behandlung befindlichen Knochen geeignet sind. Ihre Lage gibt ferner einen Hinweis darauf, ob die Einstellung der Lehre am Knochen richtig vorgenommen wurde.

Für die Fixierung der Lehre am Knochen ist ein Fixierteil vorgesehen, der sich aus zwei getrennten Seitenwangen 20 zusammensetzt. Diese haben einen Halteteil 21, der mit der Seitenfläche des frontalen Teils 16 lösbar verschraubt ist, und einen Arm 22, der in weiter unten beschriebener Weise zur Verbindung mit dem Knochen dient. Der Halteteil 21 enthält eine Längsnut 23, die einen von der Seitenfläche des frontalen Teils 16 der Sägelehre vorragenden Stift 24 im Gleitsitz aufnimmt. Sie enthält ferner fluchtend mit der Nut 23 einen Schlitz 25, durch den eine Feststellschraube 26 in die Seitenfläche des frontalen Teils 16 eindringt. Man erkennt, daß die Seitenwangen 20 bei gelöster Schraube 26 in Richtung der Nut 23 und des Schlitzes 25 verstellbar sind. Diese Richtung entspricht der Längsrichtung der Sägelehre. Diese wird so an den Oberschenkelknochen angesetzt, daß die Längsrichtung der Sägelehre mit der Richtung der Diaphyse des Knochens - abgesehen von üblichen Abweichungen - etwa übereinstimmt.

Der Arm 22 enthält Führungsbohrungen 27, 28, die je einen Fixierstift 29, 30 aufnehmen, der von der Seite her in die Condylen 1 eingeschlagen wird, um die Sägelehre in der eingestellten Position zu sichern. Die Bohrungen 27, 28 haben deutlich (d.h. um mindestens etwa 20°) voneinander abweichende Richtungen. Dadurch werden die Seitenwangen 20 und der Knochen 1 in eindeutiger Lage zueinander festgelegt. Es können auch noch mehr als die gezeigten zwei Stifte je Wange 20 vorgesehen sein. Auch können die Wangen 20 in anderer Weise ausgeführt sein. Es kommt darauf an, daß sie seitlich zu den Condylen liegen und ihre Stiftaufnahmebohrungen so angeordnet und gerichtet sind, daß die Stifte 27, 28 keine der Resektionsfacetten 2 bis 6 durchqueren oder in einem Zugangsbereich zu einer dieser Facetten liegen, in welchem die Säge gehandhabt werden muß. Zusätzlich oder statt der Stifte 29, 30 können auch andere Fixiermittel vorgesehen sein, beispielsweise Knochenschrauben oder klemmend auf den Knochen einwirkende Schrauben.

Die Sägelehre besitzt zur Einstellung und Justierung am Knochen einen lösbaren Führungsklotz 40 für einen in den Markkanal einzusetzenden Stab 41. Der Führungsklotz 40 wird von den Stangen 9b und 9c sowie der Stange 10c gehalten. Der Führungsklotz 40 befindet sich in der Mitte im Bereich des Condylenzwischenraums, in welchem kein Sägeschnitt stattfindet. Es können auch andere Mittel zur Verbindung mit dem Stab 41 vorgesehen sein, wie sie dem Fachmann bekannt sind (EP-A-661023, EP-A-243109, EP-A-709061, WO96/25114, US-A-4703751, US-A-5122144).

Wenn die Resektionsschnitte durchgeführt worden sind und das Implantat, eine Schablone oder ein Probeimplantat anprobiert werden soll, löst man die Schrauben 26 und nimmt die Sägelehre 7 von den Seitenwangen 20 ab, indem man die Schrauben 26 und die Stifte 24 durch den Schlitz 25 bzw. die Nut 23 gleiten läßt. Die Seitenwangen 20 bleiben in ihrer Position. Trotzdem geben sie den gesamten Condylenbereich frei, in welchem das Implantat oder die Schablone aufgesetzt werden muß. Stellt sich heraus, daß nachreseziert werden muß, so kann der die Säge führungen enthaltende Teil 7 der Sägelehre wieder mit den Seitenwangen 20 zusammengefügt werden. Das Auffinden der vorherigen Position bzw. die Einstellung einer von der vorherigen Position abweichenden Position kann durch Einstellskalen und Zeiger an den Halteteilen 21 der Seitenwangen 20 einerseits und des Frontteils 16 andererseits ermöglicht bzw. erleichtert werden.

Beim erstmaligen Anlegen der Sägelehre kann ein Teil derselben, beispielsweise der Führungsklotz 40 an den distalen Bereichen der Condylengleitflächen in Anlage gebracht werden. Wenn von vornherein eine stärkere Resektion beabsichtigt ist, besteht die Möglichkeit, den Anlagebereich der Sägelehre für jeden der beiden entsprechenden Condylenteile verstellbar anzuordnen.

Bei einer von der normalen Einstellung der Sägelehre an den Condylen abweichenden Einstellung verfährt man so, daß die Sägelehre zunächst einmal in normaler Stellung eingestellt wird. Danach wird sie unter Zuhilfenahme der an den Seitenwangen oder der Frontplatte 16 angebrachten Skala um das gewünschte Maß verstellt.

## Patentansprüche

1. Sägelehre für die Facettenresektion von Kniegelenk-Femurcondylen mit einer auf die Diaphyse des Femurs auszurichtenden Längsrichtung, die zwei Seitenplatten (42, 43) aufweist, zwischen denen eine Mehrzahl von zur Führung eines Sägeblatts in sämtlichen Schnittebenen bestimmten Stangenpaaren (8b, 8c; 9b, 9c; 10b, 10c; 11b, 11c; 12b, 12c) gehalten ist und an denen Bohrungen (27, 28) zur Aufnahme von Fixierstiften (29, 30) vorgesehen sind, **dadurch gekennzeichnet, daß** die Bohrungen (27, 28) zur Aufnahme der Fixierstifte (29, 30) in besonderen Seitenwangen (20) angeordnet sind, mit denen die Seitenplatten (42, 43) über Längsführungen (23, 24, 25, 26) lösbar und in Längsrichtung verstellbar verbunden sind.

2. Sägelehre nach Anspruch 1, **dadurch gekennzeichnet, daß** eine zur Anlage an den distalen Condylenflächen des Femurs bestimmte Anschlageinrichtung (40) der Sägelehre (7) in Längsrichtung verstellbar oder entfernbar ist.

3. Sägelehre nach Anspruch 1 oder 2, **dadurch gekennzeichnet, daß** sämtliche Stangen (8b, 8c; 9b, 9c; 10b, 10c; 11b, 11c; 12b, 12c) ein Längen/Durchmesser-Verhältnis von mindestens 10 haben.

## Claims

1. A saw jig for the facet resection of knee joint femur condyles having a longitudinal direction aligned to the diaphysis of the femur, which has two side plates (42,43), between which are retained a plurality of rod pairs (8b,8c; 9b,9c; 10b,10c; 11b,11c; 12b,12c) intended for guiding a saw blade in all cutting planes and in which bores (27,28) are provided for accommodating locating pins (29,30), **characterised in that** the bores (27,28) for accommodating the locating pins (29,30) are disposed in special side cheeks (20), with which the side walls (42,43) are releasably connected via longitudinal guides (23,24,25, 26) and are adjustably connected in longitudinal direction.

2. A saw jig according to Claim 1, **characterised in that** a stop means (40) of the saw jig (7), intended for abutment on the distal condyle surfaces of the femur, can be adjusted or removed in longitudinal direction.

3. A saw jig according to Claim 1 to 2, **characterised in that** all the rods (8b,8c; 9b,9c; 10b,10c; 11b,11c; 12b,12c) have a length/diameter ratio of at least 10.

## Revendications

1. Gabarit de sciage pour la résection de facettes de condyles de femur à l'articulation du genou avec une direction longitudinale à orienter vers la diaphyse du fémur, qui comporte deux plaques latérales (42, 43) entre lesquelles est maintenue une pluralité de paires de tiges (8b, 8c ; 9b, 9c ; 10b, 10c; 11b, 11c ; 12b, 12c) destinées à guider une lame de scie dans tous les plans de coupe, et sur lesquelles sont prévus des perçages (27, 28) destinés à recevoir des broches de fixation (29, 30), **caractérisé en ce que** les perçages (27, 28) destinés à recevoir les broches de fixation (29, 30) sont disposés dans des joues latérales (20) particulières auxquelles les plaques latérales (42, 43) sont reliées, par des guides longitudinaux (23, 24, 25, 26), de manière séparable et réglable dans la direction longitudinale.

2. Gabarit de sciage selon la revendication 1, **caractérisé en ce qu'**un dispositif de butée (40), destiné à s'appliquer contre les surfaces distales du condyle fémoral, du gabarit de sciage (7), peut être réglé dans la direction longitudinale ou enlevé.

3. Gabarit de sciage selon la revendication 1 ou 2, **caractérisé en ce que** toutes les tiges (8b, 8c ; 9b, 9c ; 10b, 10c ; 11b, 11c ; 12b, 12c) ont un rapport longueur/diamètre d'au moins 10.
